# EUROPEAN PATENT APPLICATION

(11) **EP 3 944 863 A1**
(43) Date of publication of application: **02.02.2022**
(21) Application number: 20382681.3
(22) Date of filing: 28.07.2020
(51) Int. Cl.: A61K 39/395, A61K 48/00, A61P 35/00, C07K 14/705, C07K 16/28, C12Q 1/6886, G01N 33/574, G01N 33/68

(54) **COMBINED THERAPY AGAINST CANCER**

(71) Applicant: Fundació Institut Hospital Del Mar D'Investigacions Mèdiques (IMIM), 08003 Barcelona (ES)
(72) Inventor: CELIÀ TERRASSA, Antoni, 08003 Barcelona (ES); ALBANELL MESTRES, Joan, 08003 Barcelona (ES); ROZALÉN MIRALLES, Catalina, 08003 Barcelona (ES); PÉREZ NÚÑEZ, Iván, 08003 Barcelona (ES)
(74) Representative: Elzaburu S.L.P.

(57) **Abstract**

The present invention relates to a pharmaceutical composition comprising a LCoR activator and an immune checkpoint inhibitor (ICI), to its use in the treatment of cancer and to the use of LCoR as marker of response to immunotherapy in cancer treatment.

SEQUENCE LISTING

<110> Fundació Institut Hospital del Mar d'Investigacions
      Mèdiques (IMIM)

<120> Combined therapy against cancer

<130> EP-2397

<160> 3

<170> PatentIn version 3.5

<210> 1
<211> 433
<212> PRT
<213> Homo sapiens

<400> 1

Met Gln Arg Met Ile Gln Gln Phe Ala Ala Glu Tyr Thr Ser Lys Asn
1               5                   10                  15

Ser Ser Thr Gln Asp Pro Ser Gln Pro Asn Ser Thr Lys Asn Gln Ser
            20                  25                  30

Leu Pro Lys Ala Ser Pro Val Thr Thr Ser Pro Thr Ala Ala Thr Thr
        35                  40                  45

Gln Asn Pro Val Leu Ser Lys Leu Leu Met Ala Asp Gln Asp Ser Pro
    50                  55                  60

Leu Asp Leu Thr Val Arg Lys Ser Gln Ser Glu Pro Ser Glu Gln Asp
65                  70                  75                  80

Gly Val Leu Asp Leu Ser Thr Lys Lys Ser Pro Cys Ala Gly Ser Thr
                85                  90                  95

Ser Leu Ser His Ser Pro Gly Cys Ser Ser Thr Gln Gly Asn Gly Arg
            100                 105                 110

Pro Gly Arg Pro Ser Gln Tyr Arg Pro Asp Gly Leu Arg Ser Gly Asp
        115                 120                 125

Gly Val Pro Pro Arg Ser Leu Gln Asp Gly Thr Arg Glu Gly Phe Gly
    130                 135                 140

His Ser Thr Ser Leu Lys Val Pro Leu Ala Arg Ser Leu Gln Ile Ser
145                 150                 155                 160

Glu Glu Leu Leu Ser Arg Asn Gln Leu Ser Thr Ala Ala Ser Leu Gly
                165                 170                 175

Pro Ser Gly Leu Gln Asn His Gly Gln His Leu Ile Leu Ser Arg Glu
        180                 185                 190

Ala Ser Trp Ala Lys Pro His Tyr Glu Phe Asn Leu Ser Arg Met Lys
    195                 200                 205

Phe Arg Gly Asn Gly Ala Leu Ser Asn Ile Ser Asp Leu Pro Phe Leu
    210                 215                 220

Ala Glu Asn Ser Ala Phe Pro Lys Met Ala Leu Gln Ala Lys Gln Asp
225                 230                 235                 240

Gly Lys Lys Asp Val Ser His Ser Ser Pro Val Asp Leu Lys Ile Pro
                245                 250                 255

Gln Val Arg Gly Met Asp Leu Ser Trp Glu Ser Arg Thr Gly Asp Gln
            260                 265                 270

Tyr Ser Tyr Ser Ser Leu Val Met Gly Ser Gln Thr Glu Ser Ala Leu
        275                 280                 285

Ser Lys Lys Leu Arg Ala Ile Leu Pro Lys Gln Ser Arg Lys Ser Met
    290                 295                 300

Leu Asp Ala Gly Pro Asp Ser Trp Gly Ser Asp Ala Glu Gln Ser Thr
305                 310                 315                 320

Ser Gly Gln Pro Tyr Pro Thr Ser Asp Gln Glu Gly Asp Pro Gly Ser
                325                 330                 335

Lys Gln Pro Arg Lys Lys Arg Gly Arg Tyr Arg Gln Tyr Asn Ser Glu
            340                 345                 350

Ile Leu Glu Glu Ala Ile Ser Val Val Met Ser Gly Lys Met Ser Val
        355                 360                 365

Ser Lys Ala Gln Ser Ile Tyr Gly Ile Pro His Ser Thr Leu Glu Tyr
    370                 375                 380

Lys Val Lys Glu Arg Leu Gly Thr Leu Lys Asn Pro Pro Lys Lys Lys
385                 390                 395                 400

Met Lys Leu Met Arg Ser Glu Gly Pro Asp Val Ser Val Lys Ile Glu
                405                 410                 415

Leu Asp Pro Gln Gly Glu Ala Ala Gln Ser Ala Asn Glu Ser Lys Asn
            420                 425                 430

Glu

<210>  2
<211>  21
<212>  PRT
<213>  Homo sapiens

<220>
<221>  VARIANT
<222>  (6)..(6)
<223>  Ser or Gly

<220>
<221>  VARIANT
<222>  (10)..(10)
<223>  Ile or Val

<400>  2

Val Ser Lys Ala Gln Xaa Ile Tyr Gly Xaa Pro His Ser Thr Leu Glu
1               5                   10                  15

Tyr Lys Val Lys Glu
            20

<210>  3
<211>  602
<212>  PRT
<213>  Homo sapiens

<400>  3

Met Asp Lys Gly Arg Glu Arg Met Ala Ala Ala Ala Ala Ala Ala Ala
1               5                   10                  15

Ala Ala Ala Ala Ala Ala Gln Cys Arg Ser Pro Arg Cys Ala Ala Glu
            20                  25                  30

Arg Arg Gly Phe Arg Arg Glu Leu Asp Ser Trp Arg His Arg Leu Met
        35                  40                  45

His Cys Val Gly Phe Glu Ser Ile Leu Glu Gly Leu Tyr Gly Pro Arg
    50                  55                  60

Leu Arg Arg Asp Leu Ser Leu Phe Glu Asp Cys Glu Pro Glu Glu Leu
65                  70                  75                  80

Thr Asp Trp Ser Met Asp Glu Lys Cys Ser Phe Cys Asn Leu Gln Arg
                85                  90                  95

Glu Ala Val Ser Asp Cys Ile Pro Ser Leu Asp Ser Ser Gln Ser Thr
            100                 105                 110

Pro Thr Glu Glu Leu Ser Ser Gln Gly Gln Ser Asn Thr Asp Lys Ile
        115                 120                 125

Glu Cys Gln Ala Glu Asn Tyr Leu Asn Ala Leu Phe Arg Lys Lys Asp
    130                 135                 140

Leu Pro Gln Asn Cys Asp Pro Asn Ile Pro Leu Val Ala Gln Glu Leu
145                 150                 155                 160

Met Lys Lys Met Ile Arg Gln Phe Ala Ile Glu Tyr Ile Ser Lys Ser
                165                 170                 175

Gly Lys Thr Gln Glu Asn Arg Asn Gly Ser Ile Gly Pro Ser Ile Val
            180                 185                 190

Cys Lys Ser Ile Gln Met Asn Gln Ala Glu Asn Ser Leu Gln Glu Glu
        195                 200                 205

Gln Glu Gly Pro Leu Asp Leu Thr Val Asn Arg Met Gln Glu Gln Asn
    210                 215                 220

Thr Gln Gln Gly Asp Gly Val Leu Asp Leu Ser Thr Lys Lys Thr Ser
225                 230                 235                 240

Ile Lys Ser Glu Glu Ser Ser Ile Cys Asp Pro Ser Ser Glu Asn Ser
                245                 250                 255

Val Ala Gly Arg Leu His Arg Asn Arg Glu Asp Tyr Val Glu Arg Ser
            260                 265                 270

Ala Glu Phe Ala Asp Gly Leu Leu Ser Lys Ala Leu Lys Asp Ile Gln
        275                 280                 285

Ser Gly Ala Leu Asp Ile Asn Lys Ala Gly Ile Leu Tyr Gly Ile Pro
    290                 295                 300

Gln Lys Thr Leu Leu Leu His Leu Glu Ala Leu Pro Ala Gly Lys Pro
305                 310                 315                 320

Ala Ser Phe Lys Asn Lys Thr Arg Asp Phe His Asp Ser Tyr Ser Tyr
                325                 330                 335

Lys Asp Ser Lys Glu Thr Cys Ala Val Leu Gln Lys Val Ala Leu Trp
            340                 345                 350

Ala Arg Ala Gln Ala Glu Arg Thr Glu Lys Ser Lys Leu Asn Leu Leu
        355                 360                 365

Glu Thr Ser Glu Ile Lys Phe Pro Thr Ala Ser Thr Tyr Leu His Gln
    370                 375                 380

Leu Thr Leu Gln Lys Met Val Thr Gln Phe Lys Glu Lys Asn Glu Ser
385                 390                 395                 400

Leu Gln Tyr Glu Thr Ser Asn Pro Thr Val Gln Leu Lys Ile Pro Gln
                405                 410                 415

Leu Arg Val Ser Ser Val Ser Lys Ser Gln Pro Asp Gly Ser Gly Leu
            420                 425                 430

Leu Asp Val Met Tyr Gln Val Ser Lys Thr Ser Ser Val Leu Glu Gly
        435                 440                 445

Ser Ala Leu Gln Lys Leu Lys Asn Ile Leu Pro Lys Gln Asn Lys Ile
    450                 455                 460

Glu Cys Ser Gly Pro Val Thr His Ser Ser Val Asp Ser Tyr Phe Leu
465                 470                 475                 480

His Gly Asp Leu Ser Pro Leu Cys Leu Asn Ser Lys Asn Gly Thr Val
                485                 490                 495

Asp Gly Thr Ser Glu Asn Thr Glu Asp Gly Leu Asp Arg Lys Asp Ser
            500                 505                 510

Lys Gln Pro Arg Lys Lys Arg Gly Arg Tyr Arg Gln Tyr Asp His Glu
        515                 520                 525

Ile Met Glu Glu Ala Ile Ala Met Val Met Ser Gly Lys Met Ser Val
    530                 535                 540

Ser Lys Ala Gln Gly Ile Tyr Gly Val Pro His Ser Thr Leu Glu Tyr
545                 550                 555                 560

Lys Val Lys Glu Arg Ser Gly Thr Leu Lys Thr Pro Pro Lys Lys Lys
                565                 570                 575

Leu Arg Leu Pro Asp Thr Gly Leu Tyr Asn Met Thr Asp Ser Gly Thr
            580                 585                 590

Gly Ser Cys Lys Asn Ser Ser Lys Pro Val
        595                 600

## Description

### Field of the invention

The present invention belongs to the field of Biotechnology and relates to a combined therapy against cancer, particularly to a pharmaceutical composition comprising a LCoR activator and an immune checkpoint inhibitor.

### Background of the invention

LCoR (Ligand dependent nuclear receptor corepressor) was first identified as a ligand-dependent interaction partner of nuclear receptors, such as estrogen receptor (ER) requiring the LXXLL of the LCoR motif for binding to the ER and other nuclear receptors (Fernandes, I et al. (2003) Mol. Cell 11, 139-150).

LCoR is a transcriptional repressor of ligand-activated ERs and other transcription factors that acts both by recruiting histone deacetylases and C-terminal binding proteins (CtBP). It has 4 variants and 3 isoforms that contain the HTH domain. The protein has an HTH domain that is a putative DNA binding site identified as relevant for transcriptomic activation (Celià-Terrassa et al. (2017) Nat Cell Biol. 19(6): 711-723). In breast cancer cell lines and tissues, two mRNAs variants of LCoR were detected, LCoR and LCoR2, the latter encoding a truncated LCoR protein (Jalaguier, S. et al. (2017) Oncogene 36, 4790-4801). LCoR and LCoR2 strongly repressed transcription, inhibited estrogen-induced target gene expression and decreased breast cancer cell proliferation. Receptor-interacting protein of 140 kDa (RIP140) was found to be a LCoR and LCoR2 partner and their expression correlated with overall survival of patients with breast cancer (Jalaguier, S. et al. (2017) Oncogene 36, 4790-4801). Celià-Terrassa et al. disclose that the miR-199a-LCoR axis mainly influences tumorigenesis of ER⁻ breast cancer, suggesting that LCoR is not acting through ER binding, but activating IFN signalling through the HTH domain acting as a tumor suppressor (Celià-Terrassa et al. (2017) Nat Cell Biol. 19(6): 711-723).

In addition, LCoR has been described to act as an androgen-dependent corepressor that represses human prostate cancer growth *in vivo.* LCoR interacts with the androgen receptor (AR) and is recruited to chromatin in an androgen-induced manner. This interaction of LCoR with AR is inhibited by signalling pathways that are associated with androgen-independent prostate cancer (Asim, M. et al. (2011) J. Biol. Chem. Vol. 286, No. 43, pp. 37108-37117). LCoRL (Ligand dependent nuclear receptor corepressor-like) also contains a similar HTH domain as LCoR.

WO2019232542 A2 relates to gene signatures for the treatment and prognosis of cancer, to be detected in the extracellular fluids, such as cerebrospinal fluid, of a subject suffering from brain metastases. These signatures are used to predict the response to immunotherapy. WO2019232542 A2 discloses that elevated levels of LCoR are related to non-responding subjects to immunotherapy.

### Description of the invention

The present invention provides a novel and improved cancer therapy based on the combination of an LCoR activator and at least one Immune checkpoint inhibitor (ICI). Moreover, the present invention also provides for a prognostic tool useful in the determination of subjects respondent to immunotherapy, particularly to ICI.

The inventors of the present invention have surprisingly found that activating LCoR increases the efficacy of the response to ICI cancer treatment. Also, the inventors have found that LCoR is a useful biomarker for predicting the response of a subject afflicted from cancer to ICI immunotherapy.

Thus, a first subject of the present invention is a pharmaceutical composition comprising:
a. an LCoR activator; and
b. at least one immune checkpoint inhibitor (ICI).

As used herein, LCoR refers to either LCoR or its truncated protein LCoR2 or to LCoRL. LCoR can also be referred to as LCOR. As used herein, the term "LCoR activator" refers to activators of LCoR, LCoR2 or LCoRL gene expression or of the expression of a nucleic acid codifying for SEQ ID NO: 1 or SEQ ID NO: 3, which are the amino acid sequences of human LCoR and LCoRL, respectively. As used herein, the term "LCoR activator" also refers to agents that increase the amount of LCoR protein, of LCoR2 protein or of LCoRL protein, or the amount of a protein comprising SEQ ID NO: 1. As used herein, the term "LCoR activator" also refers to activators of the expression of a nucleic acid codifying for SEQ ID NO: 2, which is the sequence of the HTH domain of LCoR/LCoRL. As used herein, the term "LCoR activator" also refers to agents that increase the amount of a protein comprising SEQ ID NO: 2.

Immune checkpoint inhibitors include agents that inhibit CTLA-4, PD-1, PD-L1, Lag-3, Tim-3, TIGIT and the like. Suitable anti-CTLA-4 therapy agents for use in the present invention, include, without limitation, anti-CTLA-4 antibodies, human anti-CTLA-4 antibodies, mouse anti-CTLA-4 antibodies, mammalian anti-CTLA-4 antibodies, humanized anti-CTLA-4 antibodies, monoclonal anti-CTLA-4 antibodies, polyclonal anti-CTLA-4 antibodies, chimeric anti-CTLA-4 antibodies, ipilimumab, tremelimumab, anti-CD28 antibodies, anti-CTLA-4 adnectins, anti-CTLA-4 domain antibodies, single chain anti-CTLA-4 fragments, heavy chain anti-CTLA-4 fragments, light chain anti- CTLA-4 fragments, inhibitors of CTLA-4 that agonize the co-stimulatory pathway. Suitable anti-PD-1 and anti-PD-L1 therapy agents for use in the present invention, include, without limitation, anti-PD-1 and anti-PD-L1 antibodies, human anti- PD-1 and anti-PD-L1 and anti-PD-L1 antibodies, mouse anti-PD-1 and anti-PD-L1 antibodies, mammalian anti-PD-1 and anti-PD-L1 antibodies, humanized anti-PD-1 and anti-PD-L1 antibodies, monoclonal anti-PD-1 and anti-PD-L1 antibodies, polyclonal anti-PD-1 and anti-PD-L1 antibodies, chimeric anti-PD-1 and anti-PD-L1 antibodies. In specific embodiments, anti-PD-1 therapy agents include nivolumab, pembrolizumab, pidilizumab, MEDI0680, and combinations thereof. In other specific embodiments, anti- PD-L1 therapy agents include atezolizumab, BMS-936559, MEDI4736, MSB0010718C, and combinations thereof. Suitable anti-Lag-3 therapy agents for use in the present invention include, without limitation, BMS-986016 and TSR-033. Suitable anti-Tim-3 therapy agents for use in the present invention include, without limitation, TSR-022 (cobolimab). Suitable anti-TIGIT therapy agents for use in the present invention include, without limitation, BMS-986207.

In a preferred embodiment of the composition of the first aspect, the LCoR activator is an agent that induces LCoR expression, or an agent that increases the amount of LCoR. In a preferred enbodiment, the LCoR activator is a nucleic acid comprising a codifying sequence for SEQ ID NO: 1, or for SEQ ID NO: 2, or for SEQ ID NO: 3. In another preferred embodiment of the composition of the first aspect, the LCoR activator is doxorubicin. The inventors have shown that doxorubicin increases LCoR expression when administered to cancer cells.

In a preferred embodiment of the composition of the first aspect, the at least one ICI inhibits CTLA-4, PD-1, PD-L1, Lag-3, Tim-3, TIGIT or a combination thereof, preferably inhibits PD-1 or PD-L1, more preferably inhibits PD-1. In a preferred embodiment, the ICI is selected between ipilimumab, tremelimumab, nivolumab, dostarlimab, pembrolizumab, pidilizumab, MEDI0680, atezolizumab, BMS-936559, MEDI4736 (durvalumab), MSB0010718C (avelumab), TSR-022 (cobolimab), BMS-986016, TSR-033, BMS-986207, and combinations thereof, preferably is selected between pembrolizumab, atezolizumab, dostarlimab and combinations thereof. In a preferred embodiment, the at least one ICI is dostarlimab. In a preferred embodiment, the at least one ICI is pembrolizumab. In a preferred embodiment, the at least one ICI is atezolizumab.

A second aspect of the present invention is the use of the composition of the first aspect in the treatment of cancer. Preferably, the cancer is selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably triple-negative breast cancer. In a preferred embodiment, the cancer is breast cancer. More preferably, the cancer is triple-negative breast cancer.

In a preferred embodiment of the second aspect, the LCoR activator and the ICI are administered simultaneously or sequentially. Combination treatments involving an LCoR activator and an immune checkpoint inhibitor can be achieved by administering the LCoR activator and the immune checkpoint inhibitor at the same time. Such combination treatments can be achieved by administering a single pharmaceutical composition that includes both agents, or by administering two distinct pharmaceutical compositions, at the same time, wherein one composition includes the LCoR activator and the other includes the immune checkpoint inhibitor. Alternatively, treatment with an LCoR activator can precede or follow treatment with the immune checkpoint inhibitor by intervals ranging from minutes to weeks.
In a preferred embodiment, the composition for use of the second aspect of the invention is administered before or after surgery.

A third aspect of the present invention is a prognostic method of the response to immunotherapy in a subject afflicted from cancer comprising:
(a) determining the presence of LCoR, LCoR expression levels and/or amount of LCoR protein in a biological sample from said subject,
(b) determining the presence of LCoR, LCoR expression levels and/or amount of LCoR protein in a control,
(c) comparing the presence of LCoR, LCoR expression levels and/or amount of LCoR protein determined in steps (a) and (b),
wherein the presence of LCoR versus its absence or higher LCoR expression levels or amount of LCoR protein in the subject biological sample relative to the control indicates that the subject afflicted from cancer would respond to immunotherapy.

As used herein, immunotherapy is cancer immunotherapy, particularly ICI therapy that targets immune checkpoints to restore the immune system function. Preferably, the immunotherapy comprises the therapy with at least one ICI that inhibits CTLA-4, PD-1, PD-L1, Lag-3, Tim-3, TIGIT or a combinations thereof, preferably the at least one ICI inhibits PD-1 or PD-L1, more preferably it inhibits PD-1. Preferably, the immunotherapy comprises the treatment with at least one of the following ICIs: ipilimumab, tremelimumab, nivolumab, dostarlimab, pembrolizumab, pidilizumab, MEDI0680, atezolizumab, BMS-936559, MEDI4736 (durvalumab), MSB0010718C (avelumab), TSR-022 (cobolimab), BMS-986016, TSR-033, BMS-986207, and combinations thereof, preferably the immunotherapy is selected between pembrolizumab, atezolizumab and dostarlimab.

In a preferred embodiment of the prognostic method of the third aspect, the biological sample is from the cancerous tissue of the subject afflicted from cancer. Preferably, the sample is a primary melanoma tumor sample or a bladder cancer sample.

In a preferred embodiment of the prognostic method of the third aspect, the expression levels of LCoR or the amount of LCoR protein are determined and compared in steps (a) to (c). Preferably, the expression levels of LCoR or the amount of LCoR protein are determined by qPCR, *in situ* hybridization, Western blot or immunohistochemistry (IHC).

In a preferred embodiment of the prognostic method of the third aspect, the immunotherapy is the administration of at least one ICI selected from CTLA-4, PD-1, PD-L1, Lag-3, Tim-3, TIGIT or a combination thereof, preferably the at least one ICI inhibits PD-1 or PD-L1, more preferably it inhibits PD-1. Preferably, the immunotherapy comprises the treatment with at least one of the following ICIs: ipilimumab, tremelimumab, nivolumab, dostarlimab, pembrolizumab, pidilizumab, MEDI0680, atezolizumab, BMS-936559, MEDI4736 (durvalumab), MSB0010718C (avelumab), TSR-022 (cobolimab), BMS-986016, TSR-033, BMS-986207, and combinations thereof, preferably the immunotherapy is selected between pembrolizumab, atezolizumab and dostarlimab.

In a preferred embodiment of the prognostic method of the third aspect, the cancer is selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably triple-negative breast cancer.

### Brief description of the drawings

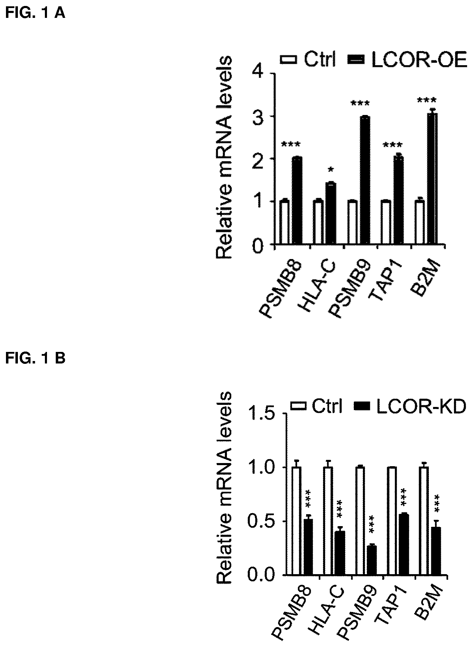

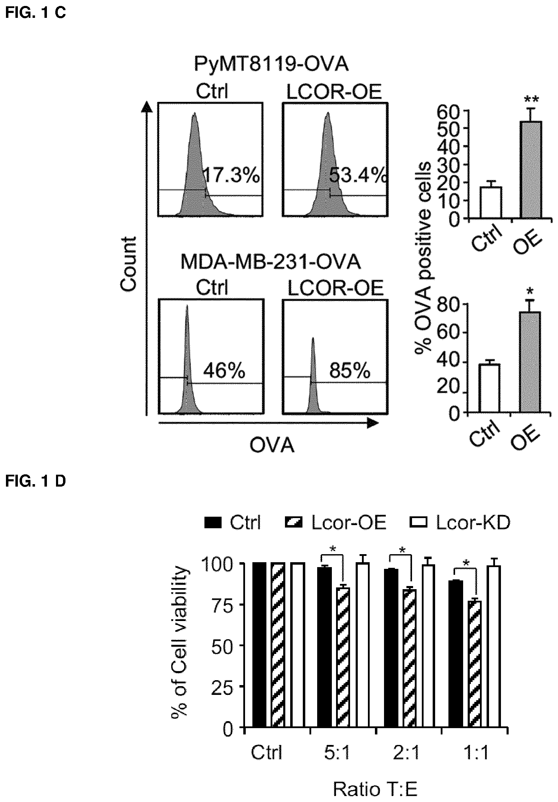

**Figure 1****.** LCoR induces the antigen processing machinery and antigen presentation in breast cancer cells. **A.** Relative mRNA levels of PSMB-8, HLA-C, PSMB-9, TAP1 and B2M in control and MDA-MB-231 breast cancer cells overexpressing LCoR. **B.** Relative mRNA levels of PSMB-8, HLA-C, PSMB-9, TAP1 and B2M in control and MDA-MB-231 breast cancer cells where LCoR was knocked down. **C.** Flow cytometry analysis of OVA in PyMT8119-OVA cells and human breast cancer MDA-MB-231-OVA cells with ectopic LCoR-OE increased the OVA positive cells **D.** The cell viability of PyMT8119-OVA cells co-cultured with CD8+ T-cells is reduced with LCoR-OE and prevented with LCoR-KD.

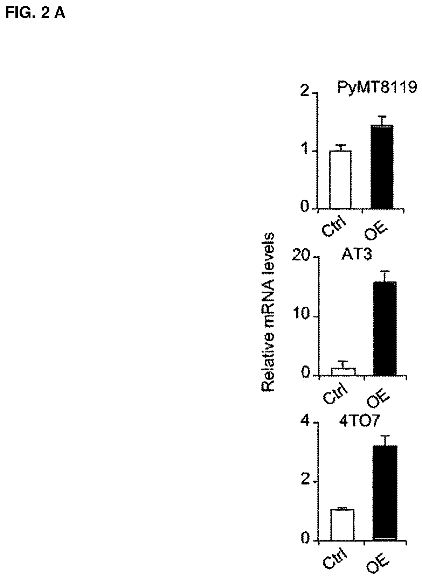

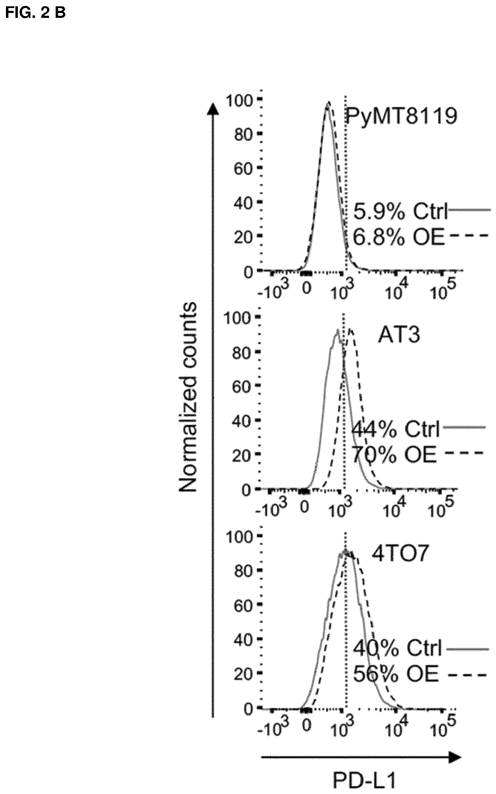

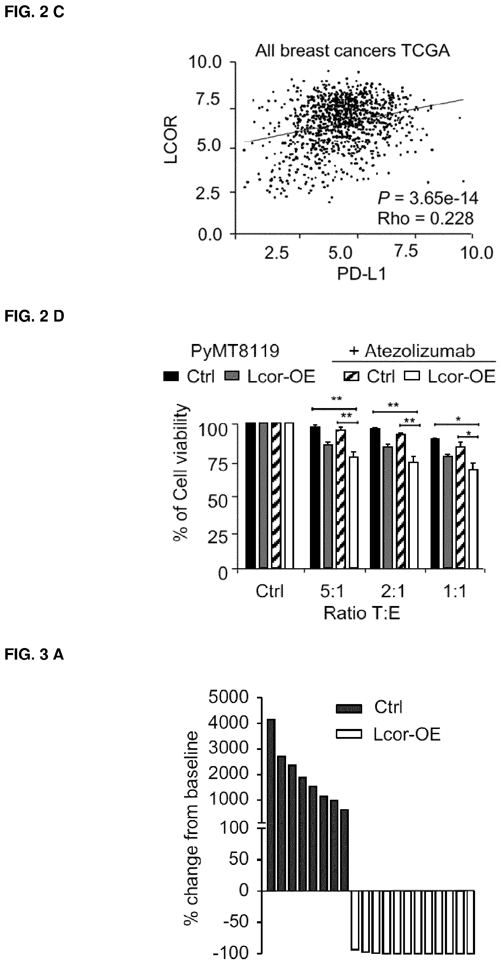

**Figure 2****.** LCoR induces PDL1 expression and T-cell killing of LCoR expressing cells in combination with immunotherapy. **A.** Relative mRNA levels of PD-L1 in the indicated mouse breast cancer cells, control and with ectopic LCoR-OE. **B.** Flow cytometry analysis of PD-L1 in the indicated mouse breast cancer cells, control and with ectopic LCoR-OE. **C.** Correlation analysis of LCoR and PD-L1 mRNA levels in 1200 breast cancer patients of the TCGA database. **D.** Cytotoxic T-cell lymphocyte (CTL) assay cocultured with PyMT8119 mouse breast cancer cells at the indicated ratios (T: tumor; E: effector T cell). Control and LCoR overexpression conditions are compared for viability with/out anti-PD-L1 treatment (Atezolizumab).

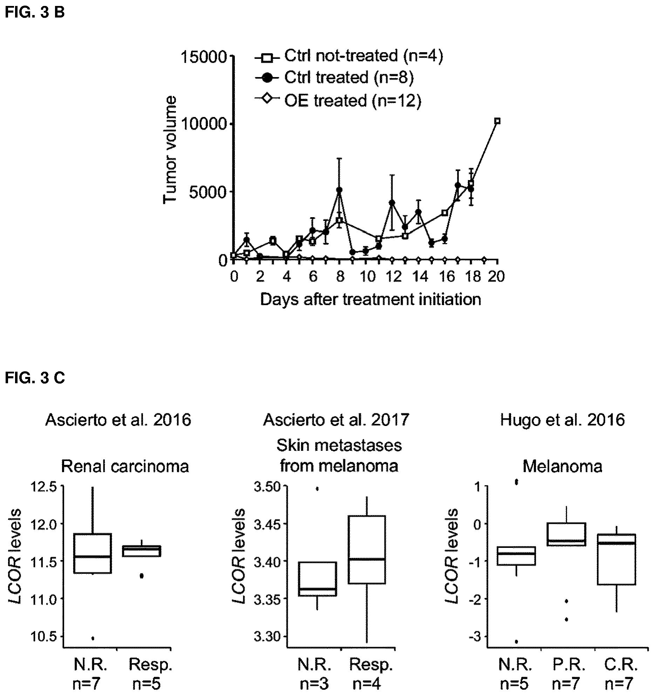

**Figure 3****.** LCoR expression induce and predicts immunotherapy response. **A.** Waterfall plot of *in vivo* tumor growth in 4TO7 mouse breast cancer cells, control and LCoR overexpression conditions treated with anti-PD-L1 (Atezolizumab). Represents the percentual change of tumor growth upon onset of the treatment until day 20. **B.** Growth curves of the same experiment in A **C.** mRNA LCoR levels comparing patients that do not respond to ICI and patients that respond in renal carcinoma and melanoma of the indicated studies.

### Examples

The following examples are provided to further illustrate, but not to limit this invention.

### Example 1. LCoR increased antigen presentation in tumor cells

We analyzed the transcriptomic profiles ectopic LCoR overexpression (OE), and LCoR knock-down (KD) in MDA-MB-231 breast cancer cells (Figs. 1 A and B). We confirmed by qPCR that AMP genes are also upregulated in breast cancer MDA-MB-231 cells overexpressing LCoR (Fig. 1 A).

We used different breast cancer cell lines transduced with the pLEX-OVA-IRES-mCherry (pLEX-OVAiC) plasmid, that overexpress the ovalbumin (OVA). The intracellular proteolytic processing of OVA generates the OVA derived peptide SIINFEKL (OVA 257-264) that is presented in the context of MHC class-I molecule H2Kb specific of the C57BL/6 background. We used this system to measure the antigen presentation ability of tumor cells by fluorescence-activated cell sorting (FACS) with a specific anti-SIINFEKL antibody.

PyMT8119-OVA cells with ectopic LCoR-OE increased the OVA positive cells from 18 % to 53 % of cells (Fig. 1 C). In human breast cancer MDA-MB-231 cells transduced with the pLEX-OVAiC, we also observed a strong increase the OVA presentation (Fig. 1 C). These results show that LCoR strongly induces cellular immunogenicity.

We also found higher immune detection and killing promoted by LCoR: we performed cytotoxic Tcell lymphocyte assays (CTL) by co-culture of CD8+ T-cells with tumor cell assays *in vitro.* The transgenic OT-I mouse model strain contain the specific TCR to detect SIINFEKL MHC-I presented. PyMT8119-OVA cells were cocultured with previously activated CD8 T-cells isolated from the spleen of OT-I mice in a 0.5 ratio of effector T-cells:tumor cells (E:T). Tumor cells with LCoR-OE were more sensitive to the immune detection and killing than the control cells, instead the LCoR-KD cells were not killed (Fig. 1 D).

### Example 2. Doxorubicin treatment in cancer cells increased LCoR expression.

Low dose treatment of breast cancer cells (360 nM during 48 hours) induced an increase of 2.3 fold change mRNA expression of LCoR in MDA-MB-231 cells.

### Example 3. LCoR increased PD-L1 expression in tumor cells and response to anti-PDL-L1 in vitro.

Using qPCR and FACS we observed that the overexpression of LCoR also induced the PD-L1 expression and membrane localization in different breast cancer cell lines (Figs. 2 A and B). In addition, analyzing the TCGA dataset of 1200 breast cancer samples, we observed a positive coexpression of LCoR and PD-L1 in breast cancer, reinforcing this regulatory loop as clinically relevant (Fig. 2 C).

These paradoxical effects of LCoR, increased immunogenicity vs. increased PD-L1, poses LCoR expressing cells as largely responsive cells to anti-PD-L1 immune-checkpoint blockade therapy. The CTL co-culture assay with OT-I CD8+ T-cells and LCoR-OE tumor cells in combination with anti-PD-L1 (Atezolizumab) showed an enhanced sensitivity to immune detection and killing (Fig. 2 D), proving that LCoR is an ideal therapeutic partner of anti-PD-L1 agents.

### Example 4. LCoR leads to complete tumor regression in combination with anti-PD-L1 therapy in vivo

The therapeutic effect of the combination of LCoR with anti-PD-L1 therapy in preclinical settings was tested as follows: we performed mammary fat pad (MFP) injections of mouse 4TO7 breast cancer cells and let the tumors grow to 0.5 cm of diameter. Strikingly, 20 out of 21 4TO7 tumor with ectopic LCoR expression completely disappeared with a regime of twice a week anti-PD-L1 treatment. Instead, none of the wild type 4TO7 tumors regressed, 20 out 20 wt tumors progressed and mice were sacrificed once the tumor burden limit was reached (Fig. 3 A). Remarkably, these 4TO7 control cells were not responsive to anti-PD-L1 therapy (Fig. 3 B), indicating that LCoR ectopic expression converted them from immunotherapy resistant state to immunotherapy sensitive.

To further exploit the potential therapeutic effects of this combination, luciferase labeled 4TO7 cells (4TO7- luc) were injected through the tail-vein to generate lung metastasis, monitored by bioluminescence (BLI) corresponding to luciferase positive cells. After 2 weeks of injection, mice with developed lung metastasis determined by BLI, were treated with anti-PD-L1 twice a week. Remarkably, metastasis of 4TO7-LCoROE cells were totally regressed and cured. However, the 4TO7-wt metastasis progressed normally until the mice were sacrificed. Therefore, we were able to cure experimental primary breast cancer and advanced metastatic breast cancer with the combination of ectopic LCoR expression and anti-PD-L1 treatment in preclinical settings.

### Example 5. LCoR correlates with immunotherapy response in different cancers

We analyzed all the clinical public available datasets GSE67501 (Ascierto et al. (2016) Cancer Immunol Res. 2;4(9):726-33) GSE79691 (Ascierto et al. (2017) Clin Cancer Res 3(12):3168-3180) GSE78220 (Hugo et al. (2016) Cell 165(1):35-44) of immunotherapy treated patients with mRNA gene expression data. Samples were from primary tumors of melanoma and bladder cancer patients before they were subjected to immune-based therapy. Despite the low number of patients of these datasets, we found that LCoR expression levels were increased in the responsive patients versus the non-responsive patients (Fig. 3 C). These results support that LCoR is a good predictive biomarker of immune-checkpoint blockade inhibitor response. In addition, immunohistochemistry of LCoR in human triple-negative breast cancer samples of 17 patients from different clinical trials showed a trend of LCoR to predict response to anti-PD1/PDL1, in which among the high LCoR expressing patients, 75 % responded to the treatment.

### Methods

**Animal studies.** Animal procedures were approved by the PRBB animal facility policy and La Generalitat de Catalunya; Departament de Territori i Sostenibilitat. Tumor formation experiment for orthotopic mammary fat pad (MFP) injections, 10 mice or glands were used for each experimental group and the primary tumors were monitored twice weekly by palpation. Tumors were measured by calipers for calculation of tumor volumes (p x length x width2/6). For lung colonization metastasis experiments, 4TO7 luciferase labeled cells were used for tail vein injections of 50,000 cells performed in Balb/c mice.

Development of metastases was monitored by measuring photon flux of metastatic lesions based on bioluminescence imaging (BLI) as previously described. For the anti-PD-L1 treatment experiment, subcutaneously injection of 10 mg/kg of monoclonal InVivoMab anti-mouse PD-L1 (BioXcell) once a week.

The transgenic OT-I mouse model strain with specific TCR for the OVA-derived peptide SIINFEKL MHC-I presented, were used to isolate splenocytes and CD8+ T-cells that specifically recognize the SIINFEKL peptide (OVA 257-264). 9-week OT-I mice were sacrificed to obtain the spleen. Using mechanical dissociation of the spleen over a sterile 40 µm mesh screen. Total splenocytes were plated in six well plates and non-attached T cells were separated from adherent dendritic cells (DC) after 24 hours. CD8+ T-cells were isolated by FACS.

**Cell lines, culture conditions and treatments.** All cell lines used in the study, including mammary epithelial cell lines (human HMLE), breast cancer cell lines (human MDA-MB-231 and mouse 4TO7; PyMT8119; AT3 cell lines) and other cell lines (HEK293T, H29 and HeLa), were cultured using the standard conditions according the American Type Culture Collection (ATCC) instructions. Doxorrubicin treatment was performed at 360 nM during 48 hours in MDA-MB-231 cells.

**Viral production and infection of cell lines.** Lentiviral plasmids were transfected into HEK293T cells together with the envelope plasmid (VSVG) and gag-pol plasmid (pCMV-dR8.91) following the standard lentiviral packaging protocol to generate lentiviruses. For established cell lines, cells were transduced in culture and selected with the corresponding antibiotic resistance. For ectopic expression of human and mouse LCoR, we used the previously generated overexpressing constructs pLEX-LCoR-HA and pLEXLcor (Celià-Terrassa et al. 2017), respectively. For gene knock-down studies, shRNA lentiviral vectors were purchased from Sigma-Aldrich for targeting mouse genes *Lcor* (TRCN0000085107) and human *LCoR* (TRCN0000016306 and TRCN0000436034). For OVA ectopic expression, lentiviral particles were generated using the pLEX-OVA-IRES-mCherry (pLEX-OVAiC) plasmid to overexpress the ovalbumin (OVA) in PyMT8119, HMLE, MDA-MB-231 and AT3 cells. Proteolytic processing of OVA generated the OVA-derived peptide SIINFEKL (OVA 257-264) that is presented in the context of MHC class I molecule H2Kb specific of C57BL/6 background.

**Flow cytometry analysis and cell sorting.** Cultured cells were lifted using trypsin (Gibco), resuspended in FACS buffer (PBS supplemented with 5 % newborn calf serum) and filtered through 70 mm nylon cell strainers. Samples were stained with anti- SIINFEKL-APC antibody (1:200, Cell Signaling), and anti- PD-L1-PE antibody (1:200, Cell Signaling, 3199) for 30 minutes. Cells were analyzed on a FACSort LSRII instrument (BD Biosciences) and analyzed with FlowJo vX. CD8+ T-cell isolation was performed staining with 1:500 of the anti-mouse CD8-PE antibody (Biolegend) using the FACSAria.

**Cytotoxic T-cell lymphocyte (CTL) co-culture assays.** CD8+ T-cells were isolated from the spleen of OT-I mice and activated in culture with OVA peptide during 48 hours. Activated CD8+ T-cells were cocultured in 24-well plates with PyMT8119-OVA cells in different ratios of effector T-cells:tumor cells (E:T). After 5 days of coculture, T-cells and debris were removed, washed with media and stained with Sulforhodamine B (SRB) for measuring cytotoxicity (Vichai and Kirtikara 2006). Briefly, the SRB colorimetric assay was performed by fixing the remaining cell with 10% trichloroacetic acid (TCA) and stained with SRB for 30 minutes, washed with 1% acetic acid and dissolve with 10 mM Tris-base solution. Measure OD at 510 nm with spectrometer.

**qRT-PCR analyses.** Total mRNA was isolated using the RNAeasy isolation Kit (QIAGEN). mRNA reverse transcription was done using Applied Biosystems reactants and real-time quantitative PCR performed using the Power SYBR green PCR master mix (Applied Biosystems). All analyses were performed using an ABI 7900HT PCR machine. mRNA expression was normalized by the expression of *GAPDH* in each sample.

**Immunohistochemistry.** Immunohistochemical (IHC) staining was performed at the Barcelona MAR Health Park Histopathology Core using a Dako PT link pre-treatment system with anti-LCoR (Novus Biologicals, NBP1-83477; 1:100 dilution). Nuclear immunoreactive score (nIRS) were done for LCoR valuation. Slides were visualized and analyzed using QuPath-0.2.0 software. For immune infiltration, we consider up to 1 mm of positive cells surrounding tumors. Necrotic areas were not considered in the quantification.

**Statistical analysis.** Results were represented as indicated in figure legends, generally as mean ± s.e. (standard error). For experiments with two groups, a small sample size (less than 30), and normally distributed data, the significance was evaluated using a two-tailed unpaired Student's t-test under the assumption of unequal variance. Asterisks will denote p-value significance: p<0.05*; p<0.01**; p<0.005***.

For multiple independent groups, one-way ANOVA was evaluated. Non-parametric data sets were evaluated using the Mann-Whitney-Wilcoxon U test. For correlation analysis of clinical samples, Pearson correlation was used to assess significance. All of the experiments with images (BLI and FACS) were repeated >3 times and representative images are shown.

**Analyzed datasets.** Clinical transcriptomic data of breast cancer patients (1200 patients) from TCGA dataset were download using TCGAbiolinks package in Rstudio (Colaprico A. et al 2015; Silvia, CT et al 2016; Mounir M. et al 2019). Levels of gene expression were measured by RNA-Seq by Expectation-Maximization (RSEM) and transformed to log₂(RSEM). Coexpression analysis of PD-L1 and LCOR expression was performed using Spearman's correlation. *P-value* and *Rho* coefficient was calculated.

## Claims

1. A pharmaceutical composition comprising:
a. an LCoR activator; and
b. at least one immune checkpoint inhibitor (ICI).

2. The pharmaceutical composition according to the preceding claim, wherein the LCoR activator is an agent that induces LCoR expression, or an agent that increases the amount of LCoR.

3. The pharmaceutical composition according to any one of the preceding claims, wherein the LCoR activator is doxorubicin.

4. The pharmaceutical composition according to any one of claims 1 or 2, wherein the LCoR activator is a nucleic acid comprising a codifying sequence for SEQ ID NO: 1, or for SEQ ID NO: 2 or for SEQ ID NO: 3.

5. The pharmaceutical composition according to any one of the preceding claims, wherein the at least one ICI inhibits CTLA-4, PD-1, PD-L1, Lag-3, Tim-3, TIGIT or a combination thereof, preferably inhibits PD-1 or PD-L1, more preferably inhibits PD-1.

6. The pharmaceutical composition according to any one of the preceding claims, wherein the ICI is selected between ipilimumab, tremelimumab, nivolumab, dostarlimab, pembrolizumab, pidilizumab, MEDI0680, atezolizumab, BMS-936559, MEDI4736 (durvalumab), MSB0010718C (avelumab), TSR-022 (cobolimab), BMS-986016, TSR-033, BMS-986207, and combinations thereof, preferably is selected between pembrolizumab, atezolizumab, dostarlimab and combinations thereof.

7. The composition according to any one of the preceding claims for use in the treatment of cancer.

8. The composition for use according to the preceding claim, wherein the cancer is selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably triple-negative breast cancer.

9. The composition for use according to the preceding claim, wherein the LCoR activator and the ICI are administered simultaneously or sequentially.

10. A prognostic method of the response to immunotherapy in a subject afflicted from cancer comprising:
(a) determining the presence of LCoR, LCoR expression levels and/or amount of LCoR protein in a biological sample from said subject,
(b) determining the presence of LCoR, LCoR expression levels and/or amount of LCoR protein in a control,
(c) comparing the presence of LCoR, LCoR expression levels and/or amount of LCoR protein determined in steps (a) and (b),
wherein the presence of LCoR versus its absence or higher LCoR expression levels or amount of LCoR protein in the subject biological sample relative to the control indicates that the subject afflicted from cancer would respond to immunotherapy.

11. The prognostic method according to the preceding claim, wherein the biological sample is from the cancerous tissue of the subject afflicted from cancer.

12. The prognostic method according to any one of the two preceding claims, wherein the expression levels of LCoR or the amount of LCoR protein are determined and compared in steps (a) to (c).

13. The prognostic method according to any one of the three preceding claims, wherein the expression levels of LCoR or the amount of LCoR protein are determined by qPCR, *in situ* hybridization, Western blot or immunohistochemistry (IHC).

14. The prognostic method according to any one of the four preceding claims, wherein the immunotherapy is the administration of at least one ICI selected from CTLA-4, PD-1, PD-L1, Lag-3, Tim-3, TIGIT or a combination thereof, preferably the at least one ICI inhibits PD-1 or PD-L1, more preferably it inhibits PD-1.

15. The prognostic method according to any one of the five preceding claims, wherein the cancer is selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably triple-negative breast cancer.
